# EUROPEAN PATENT SPECIFICATION

(11) **EP 0 832 229 B1**
(45) Date of publication and mention of the grant of the patent: **14.01.2004**
(21) Application number: 96921405.5
(22) Date of filing: 06.06.1996
(51) Int. Cl.: C12N 15/12, C07K 14/725, C12N 15/62, C12N 5/10

(54) **CD40L MUTEIN**
CD40L MUTEIN
MUTEINE DE CD40L

(30) Priority: 07.06.1995 US 477733; 07.06.1995 US 484624
(43) Date of publication of application: 01.04.1998
(73) Proprietor: IMMUNEX CORPORATION, Seattle Washington 98101 (US)
(72) Inventor: ARMITAGE, Richard, J., Bainbridge Island, WA 98110 (US); FANSLOW, William, C., III, Federal Way, WA 98023 (US); SPRIGGS, Melanie, K., Seattle, WA 98119 (US); SRINIVASAN, Subhashini, Kirkland, WA 98034 (US); GIBSON, Marylou, G., Carlsbad, CA 92009 (US)
(74) Representative: Cornish, Kristina Victoria Joy
(86) International application number: PCT/US1996/009632
(87) International publication number: WO 1996/040918

(56) References cited:
- EP-A- 0 585 943
- WO-A-93/08207
- WO-A-94/10308
- BIOCHEMISTRY, vol. 34, no. 6, February 1995, EASTON, PA US, pages 1833-1844, XP002019526 J. BAJORATH ET AL: "Identification of residues on CD40 and its ligand which are critical for the receptor-ligand interaction"

## Description

### BACKGROUND OF THE INVENTION

Human CD40 protein (CD40) is a peptide of 277 amino acids having a molecular weight of 30,600, and a 19 amino acid secretory signal peptide comprising predominantly hydrophobic amino acids (Stamenkovic et al., *EMBO J.* 8:1403, 1989). CD40 belongs to a family of receptors whose members include two forms of TNF receptor (Smith et al., *Science* 248:1019, 1990; and Schall et al., *Cell* 61:361, 1990), nerve growth factor receptor (Johnson et al., *Cell* 47:545, 1986), T cell antigen OX40 (Mallett et al., *EMBO J.* 9:1063, 1990), human *Fas* antigen (Itoh et al., *Cell* 66:233, 1991) and murine 4-1BB receptor (Kwon et al., *Cell. Immunol*. 121:414, 1989 [Kwon et al. I] and Kwon et al., *Proc. Natl. Acad. Sci. USA* 86:1963, 1989 [Kwon et al. II]). The molecular weight (exclusive of glycosylation) of the mature human CD40 protein is 28,300.

Activated CD4+ T cells express high levels of a ligand for CD40 (CD40L). Human CD40L, a membrane-bound glycoprotein, was cloned from peripheral blood T-cells as described in Spriggs et al., *J. Exp. Med*. 176:1543 (1992), and WO 93/08207. The cloning of murine CD40L is described in Armitage et al., *Nature* 357:80, 1992. CD40L induces B-cell proliferation in the absence of any co-stimulus, and can also induce production of immunoglobulins in the presence of cytokines. CD40L is a type II membrane polypeptide having an extracellular region at its C-terminus, a transmembrane region and an intracellular region at its N-terminus. Soluble forms of CD40L comprise the extracellular region of CD40L or a fragment thereof. CD40L biological activity is mediated by binding of the extracellular region of CD40L with CD40, and includes B cell proliferation and induction of antibody secretion (including IgE secretion).

Prior to the present invention, a ligand for CD40 that exhibited higher binding affinity for human CD40 than that of native CD40L was unknown. Accordingly, there is a need in the art to identify and characterize such a CD40 ligand mutein.

### SUMMARY OF THE INVENTION

A novel cytokine, hereafter referred to as "CD40L," was isolated and characterized as described in WO 93/08207. The present invention comprises isolated DNA molecules encoding novel human CD40L muteins, and their complements. The isolated DNA molecules are selected from the group consisting of a DNA that encodes a polypeptide having an amino acid sequence as set forth in SEQ ID NO:2 wherein the cysteine at amino acid 194 is replaced with tryptophan, and DNA's that encode a polypeptide that is a fragment of the mutein comprising tryptophan at amino acid 194.

DNA's encoding fragments of a CD40L mutein are selected from the group consisting of peptides camprising amino acids 1 through 261, 35 through 261, 34 through 225, 113 through 261, 113 through 225, 120 through 261, or 120 through 225 of SEQ ID NO:2 in which the cysteine at the amino acid corresponding to amino acid 194 of SEQ ID NO:2 is replaced with tryptophan. DNA's that hybridize to any of the foregoing DNA's under stringent conditions (hybridization in 6 X SSC at 63°C overnight; washing in 3 X SSC at 55°C), and which encode a peptide that binds to CD40 and in which the cysteine at the amino acid corresponding to amino acid 194 of SEQ ID NO:2 is replaced with tryptopban, can also be prepared.

The invention further provides novel CD40L muteins encoded by the inventive DNA molecules. The novel muteins differ from native human CD40L in having an amino acid sequence as set forth in SEQ ID NO:2 wherein the cysteine at amino acid 194 is replaced with tryptophan. The novel muteins includes fragments of the extracellular domain of CD40L that bind CD40 and that include tryptophan at amino acid 194. Preferably the fragments are selected from the group consisting of peptides comprising amino acids 1 through 261, 35 through 261, 34 through 225, 113 through 261, 113 through 225, 120 through 261, or 120 through 225 of SEQ ID NO:2, most preferably comprising amino 113 through 261, in which the cysteine at the amino acid corresponding to amino acid 194 of SEQ ID NO:2 is replaced with tryptophan.

### BRIEF DESCRIPTION OF THE DRAWINGS

Figure 1 presents the binding of trimeric human and murine CD40L and dimeric human and murine CD40L to C40/Fc as determined in a biosensor assay.
Figure 2 illustrates the binding of trimerie human CD40L (Figure 2A) and two preparations of monomeric human CD40L (Figure 2B) to C40/Fc in a biosensor assay.

### DETAILED DESCRIPTION OF THE INVENTION

Novel polypeptides that can act as ligands for murine and human CD40 were isolated and sequenced as described in WO 93/08207. The present invention provides DNA molecules encoding novel human CD40L muteins, and their complements. The isolated DNA molecules are selected from the group consisting of a DNA that encodes a polypeptide having an amino acid sequence as set forth in SEQ ID NO:2 wherein the cysteine at amino acid 194 of SEQ ID NO:2 is replaced with tryptophan and DNA's that encode fragments thereof comprising said tryptophan at position 194 in SEQ is NO:2.

CD40L is a ligand for CD40, a receptor that is a member of the TNF receptor super family. Full-length CD40L is a membrane-bound polypeptide with an extracellular region at its C terminus, a transmembrane region, and an intracellular region at its N-terminus. A soluble version of CD40L can be made from the extracellular region or a fragment thereof. The extracellular region of human CD40L extends from amino acid 47 to amino acid 261 of SEQ ID NO:1. The biological activity of CD40L is mediated by binding to CD40 and comprises proliferation of B cells and induction of immunoglobulin secretion from activated B cells.

The novel CD40L mutein described herein was obtained by mutation of nucleotide sequences coding for a CD40L polypeptide. A CD40L mutein or analog, as referred to herein, is a polypeptide substantially bomologous to a native CD40L but which has an amino acid sequence different from native sequence CD40L polypeptide because of one or a plurality of deletions, insertions or substitutions. Analogs of CD40L can be synthesized from DNA constructs prepared by oligonucleotide synthesis and ligation or by site-specific mutagenesis techniques.

Those of skill in the art will recognize that additional mutations could be made to a DNA encoding CD40L having a tryptophan at amino acid 194. Generally, substitutions should be made conservatively; i.e., the most preferred substitute amino acids are those which do not affect the ability of the inventive proteins to bind their receptors in a manner substantially equivalent to that of native CD40L. Examples of conservative substitutions include substitution of amino acids outside of the binding domain(s), and substitution of amino acids that do not alter the secondary and/or tertiary structure of CD40L. Additional examples include substituting one aliphatic residue for another, such as Ile, Val, Leu, or Ala for one another, or substitutions of one polar residue for another, such as between Lys and Arg; Glu and Asp; or Gln and Asn. Other such conservative substitutions, for example, substitutions of entire regions having similar hydrophobicity characteristics, are well known.

Similarly, when a deletion or insertion strategy is adopted, the potential effect of the deletion or insertion on biological activity should be considered. Subunits of the inventive proteins may be constructed by deleting terminal or internal residues or sequences to form fragments. Additional guidance as to the types of mutations that can be made is provided by a comparison of the sequence of CD40L to the sequences and structures of other TNF family members.

The primary amino acid structure of the inventive CD40L mutein may be modified to create CD40L derivatives by forming covalent or aggregative conjugates with other chemical moieties, such as glycosyl groups, lipids, phosphate, acetyl groups and the like, or by creating amino acid sequence mutants. Covalent derivatives of CD40L are prepared by linking particular functional groups to CD40L amino acid side chains or at the N-terminus or C-terminus of a CD40L mutein or the extracellular domain thereof.

Other derivatives of CD40L within the scope of this invention include covalent or aggregative conjugates of CD40L or its fragments with other proteins or polypeptides, such as by synthesis in recombinant culture as N-terminal or C-terminal fusions. For example, the conjugate may comprise a signal or leader polypeptide sequence at the N-terminal region or C-terminal region of a CD40L polypeptide which co-translationally or post-translationally directs transfer of the conjugate from its site of synthesis to a site inside or outside of the cell membrane or cell wall (e.g. the α-factor leader of *Saccharomyces*).

CD40L polypeptide fusions can comprise polypeptides added to facilitate purification and identification of CD40L (e.g. poly-His), or fusions with other cytokines to provide novel polyfunctional entities. Other cytokines include, for example, any of interleukins-1 through 13, TNF (tumor necrosis factor), GM-CSF (granulocyte macrophage-colony stimulating factor), G-CSF (granulocyte-colony stimulating factor), MGF (mast cell growth factor), EGF (epidermal growth factor), PDGF (platelet-derived growth factor), NGF (nerve growth factor), EPO (erythropoietin), γ-IFN (gamma interferon), 4-1BB-L (4-1BB ligand) and other cytokines that affect immune cell growth, differentiation or function.

Biological activity of CD40L may be determined, for example, by competition for binding to the ligand binding domain of CD40 (i.e. competitive binding assays). Several useful binding assays are disclosed herein. Those skilled in the art can readily apply routine experimentation to develop binding assays, using either isolated CD40 protein (i.e., CD40/Fc) or cells expressing CD40.

CD40L may also be assayed by measuring biological activity in a B cell proliferation assay. Human B cells may be obtained from human tonsils by purification by negative selection and Percoll density sedimentation, as described by Defrance et al., *J. Immunol*. 139:1135, 1987. Burkitt lymphoma cell lines may be used to measure cell proliferation in response to CD40L. Examples of Burkitt lymphoma cell lines include, for example, Raji (ATCC CCL 86), Daudi (ATCC CCL 213) and Namalwa (ATCC CRL 1432).

Yet another assay for determining CD40L biological activity is to measure immunoglobulin produced by B cells in response to activation by CD40L or a derivative or analog thereof. Polyclonal immunoglobulin secretion can be measured, for example, by incubating with 5 × 10⁵ B cells/ml in culture for at least seven days. Immunoglobulin (Ig) production can be measured by an ELISA assay such as one described in Maliszewski et al., *J. Immunol*. 144:3028, 1990 [Maliszewski et al. I] or Maliszewski et al., *Eur J. Immunol.* 20:1735, 1990 [Maliszewski et al. II].

CD40L polypeptides may exist as oligomers, such as dimers or trimers. Oligomers are linked by disulfide bonds formed between cysteine residues on different CD40L polypeptides. Alternatively, one can link two soluble CD40L domains with a linker sequence such as those described in United States Patent 5,073,627. CD40L polypeptides may also be created by expression of a fusion protein comprising soluble CD40L (extracellular domain) and an Fc region of an immunoglobulin (for example, SEQ ID NO:4) to form divalent CD40L. Fusion proteins made with both heavy and light chains of an antibody will form a CD40L oligomer with as many as four CD40L extracellular regions. Trimeric CD40L is prepared by expressing a DNA encoding a fusion protein comprising a peptide that forms a trimer in solution (for example, a mutein of a yeast GCN4 "leucine zipper" (SEQ ID NO:5), or the lung surfactant protein D (SPD) trimerization domain (SEQ ID NO:6; Hoppe, et al., *FEBS Letters* 344:191, 1994) followed by the extracellular region of CD40L.

Fusion proteins can be prepared using conventional techniques of enzyme cutting and ligation of fragments from desired sequences. PCR techniques employing synthetic oligonucleotides may be used to prepare and/or amplify the desired fragments. Overlapping synthetic oligonucleotides representing the desired sequences can also be used to prepare DNA constructs encoding fusion proteins. Fusion proteins can also comprise CD40L and two or more additional sequences, including a leader (or signal peptide) sequence, oligomerizing region (i.e., Fc region, leucine zipper moiety or suitable zipper moiety), linker sequence, and sequences encoding highly antigenic moieties that provide a means for facile purification or rapid detection of a fusion protein.

Signal peptides facilitate secretion of proteins from cells. An exemplary signal et peptide is amino acids -26 through -1 of SEQ ID NO:8. The Flag® octapeptide (Hopp et al., *Bio*/*Technology* 6:1204, 1988) does not alter the biological activity of fusion proteins, is highly antigenic and provides an epitope reversibly bound by a specific monoclonal antibody, enabling rapid detection and facile purification of the expressed fusion protein. The Flag® sequence is also specifically cleaved by bovine mucosal enterokinase at the residue immediately following the Asp-Lys pairing, fusion proteins capped with this peptide may also be resistant to intracellular degradation in *E. coli.* A murine monoclonal antibody that binds the Flag® sequence has been deposited with the ATCC under accession number HB 9259; methods of using the antibody in purification of fusion proteins comprising the Flag® sequence are described in U.S. Patent 5,011,912.

Suitable Fc regions are defined as Fc regions that can bind to protein A or protein G, or alternatively, are recognized by an antibody that can be used in purification or detection of a fusion protein comprising the Fc region. Preferable Fc regions include the Fc region of human IgG₁ or murine IgG₁. One example is the human IgG₁ Fc region shown in SEQ ID NO:4. Fragments of a suitable Fc region may also be used, for example, an Fc region of human IgG₁ from which has been deleted a sequence of amino acids responsible for binding to protein A, such that the fragment binds to protein G but not protein A.

A linker sequence that separates the extracellular region of the CD40L from the oligomerizing moiety by a distance sufficient to ensure that the CD40L properly folds into its secondary and tertiary structures may also be used. Suitable linker sequences (1) will adopt a flexible extended conformation, (2) will not exhibit a propensity for developing an ordered secondary structure which could interact with the functional domains of fusion proteins, and (3) will have minimal hydrophobic or charged character which could promote interaction with the functional protein domains. Typical surface amino acids in flexible protein regions include Gly, Asn and Ser. Virtually any permutation of amino acid sequences containing Gly, Asn and Ser would be expected to satisfy the above criteria for a linker sequence. Other near neutral amino acids, such as Thr and Ala, may also be used in the linker sequence. The length of the linker sequence may vary without significantly affecting the biological activity of the fusion protein. Linker sequences are unnecessary where the proteins being fused have non-essential N- or C-terminal amino acid regions which can be used to separate the functional domains and prevent steric interference.

DNA constructs that encode various additions or substitutions of amino acid residues or sequences, or deletions of terminal or internal residues or sequences not needed for biological activity or binding can be prepared. For example, the extracellular CD40L N-glycosylation site can be modified to preclude glycosylation while allowing expression of a homogeneous, reduced carbohydrate analog using yeast expression systems. N-glycosylation sites in eukaryotic polypeptides are characterized by an amino acid triplet Asn-X-Y, wherein X is any amino acid except Pro and Y is Ser or Thr. Appropriate modifications to the nucleotide sequence encoding this triplet will result in substitutions, additions or deletions that prevent attachment of carbohydrate residues at the Asn side chain.

Other approaches to mutagenesis involve modification of sequences encoding dibasic amino acid residues to enhance expression in yeast systems in which KEX2 protease activity is present. Sub-units of a CD40L polypeptide may be constructed by deleting sequences encoding terminal or internal residues or sequences. Moreover, other analyses may be performed to assist the skilled artisan in selecting sites for mutagenesis. For example, WO 93/08207 discusses methods of selecting ligand agonists and antagonists.

The sequence of murine CD40L cDNA was obtained by direct expression techniques; the sequence of human CD40L was obtained by cross-species hybridization techniques using the murine CD40L cDNA as a probe. Both are described in WO 93/08207. Briefly, the extracellular region of human CD40 (the receptor; SEQ ID NO:3) was cloned by polymerase chain reaction (PCR) techniques using primers based upon a sequence published in Stamenkovic et al. A CD40/Fc fusion protein was obtained by PCR techniques by fusing DNA encoding the extracellular domain of CD40 with DNA encoding the Fc domain of human IgG 1 (SEQ ID NO:4). Purified soluble CD40 proteins were able to antagonize CD40L-mediated biological activities.

A cDNA library was prepared from an EL4 cell line sorted by FACS (fluorescence activated cell sorting) on the basis of binding of a biotinylated CD40/Fc fusion protein. Cells were sorted five times until there was a significant shift in fluorescence intensity based upon expression of a ligand for CD40 by the sorted EL-4 cells. Briefly, cDNA was synthesized, inserted into empty pDC406 vector and transformed into *E. coli.* Transformants were pooled, and the DNA from the pools was isolated and transfected into CV1-EBNA cells to create an expression cloning library. Transfected CV1-EBNA cells were cultured on slides to permit transient expression of CD40L. The slides were screened with radioiodinated CD40/Fc, and examined to identify cells expressing CD40L by ascertaining the presence of autoradiographic silver grains against a light background.

A single clone was isolated and sequenced by standard techniques, to provide the cDNA sequence and deduced amino acid sequence of murine CD40L. The human homologue CD40L cDNA was found by cross species hybridization techniques. Briefly, a human peripheral blood lymphocyte (PBL) cDNA library was made from activated peripheral blood lymphocytes. A murine probe was constructed corresponding to the coding region of murine CD40L from nucleotide 13 to nucleotide 793 of murine CD40L. Using standard techniques for cross-species hybridization, the probe was used to identify a clone that expressed human CD40L. The nucleotide and amino acid sequences of human CD40L are shown in SEQ ID NO's 1 and 2.

Recombinant expression vectors for expression of CD40L by recombinant DNA techniques include a CD40L DNA sequence comprising a synthetic or cDNA-derived DNA fragment encoding a CD40L polypeptide, operably linked to a suitable transcriptional or translational regulatory nucleotide sequence, such as one derived from a mammalian, microbial, viral, or insect gene. Examples of regulatory sequences include sequences having a regulatory role in gene expression (e.g., a transcriptional promoter or enhancer), optionally an operator sequence to control transcription, a sequence encoding an mRNA ribosomal binding site, and appropriate sequences which control transcription and translation initiation and termination.

Nucleotide sequences are operably linked when the regulatory sequence functionally relates to the CD40L DNA sequence. Thus, a promoter nucleotide sequence is operably linked to a CD40L DNA sequence if the promoter nucleotide sequence controls the transcription of the CD40L DNA sequence. Still further, a ribosome binding site may be operably linked to a sequence for a CD40L polypeptide if the ribosome binding site is positioned within the vector to encourage translation. In addition, sequences encoding signal peptides can be incorporated into expression vectors. For example, a DNA sequence for a signal peptide (secretory leader) may be operably linked to a CD40L DNA sequence

Suitable host cells for expression of CD40L polypeptides include prokaryotes, yeast or higher eukaryotic cells. Prokaryotes include gram negative or gram positive organisms, for example, *E. coli* or *Bacilli.* Suitable prokaryotic host cells for transformation include, for example, *E. coli, Bacillus subtilis, Salmonella typhimurium*, and various other species within the genera *Pseudomonas, Streptomyces,* and *Staphylococcus.* Higher eukaryotic cells include established cell lines of mammalian origin. Cell-free translation systems could also be employed to produce CD40L polypeptides using RNAs derived from DNA constructs disclosed herein. Appropriate cloning and expression vectors for use with bacterial, fungal, yeast, and mammalian cellular hosts are described, for example, in Pouwels et al. *Cloning Vectors: A Laboratory Manual,* Elsevier, New York, (1985).

The expression vectors carrying the recombinant CD40L DNA sequence are transfected or transformed into a substantially homogeneous culture of a suitable host microorganism or mammalian cell line. Transformed host cells are cells which have been transformed or transfected with nucleotide sequences encoding CD40L polypeptides and express CD40L polypeptides. Expressed CD40L polypeptides will be located within the host cell and/or secreted into culture supernatant fluid, depending upon the nature of the host cell and the gene construct inserted into the host cell.

Expression vectors transfected into prokaryotic host cells generally comprise one or more phenotypic selectable markers. A phenotypic selectable marker is, for example, a gene encoding a protein that confers antibiotic resistance or that supplies an autotrophic requirement, and an origin of replication recognized by the host to ensure amplification within the host. Other useful expression vectors for prokaryotic host cells include a selectable marker of bacterial origin derived from commercially available plasmids. This selectable marker can comprise genetic elements of the cloning vector pBR322 (ATCC 37017). pBR322 contains genes for ampicillin and tetracycline resistance and thus provides simple means for identifying transformed cells. The pBR322 "backbone" sections are combined with an appropriate promoter and a CD40L DNA sequence. Other commercially available vectors include, for example, pKK223-3 (Pharmacia Fine Chemicals, Uppsala, Sweden) and pGEM1 (Promega Biotec, Madison, WI, USA).

Promoter sequences are commonly used for recombinant prokaryotic host cell expression vectors. Common promoter sequences include β-lactamase (penicillinase), lactose promoter system (Chang et al., *Nature 275*:615, 1978; and Goeddel et al., *Nature 281*:544, 1979), tryptophan (trp) promoter system (Goeddel et al., *Nucl. Acids Res. 8*:4057, 1980; and EP-A-36776) and tac promoter (Maniatis, *Molecular Cloning: A Laboratory Manual,* Cold Spring Harbor Laboratory, p. 412, 1982). A particularly useful prokaryotic host cell expression system employs a phage λ P_{L} promoter and a cI857ts thermolabile repressor sequence. Plasmid vectors available from the American Type Culture Collection which incorporate derivatives of the λ P_{L} promoter include plasmid pHUB2 (resident in *E. coli* strain JMB9 (ATCC 37092)) and pPLc28 (resident in *E. coli* RR1 (ATCC 53082)).

CD40L may be expressed in yeast host cells, preferably from the *Saccharomyces* genus (e.g., *S. cerevisiae*). Other genera of yeast, such as *Pichia* or *Kluyveromyces,* may also be employed. Yeast vectors will often contain an origin of replication sequence from a 2µ yeast plasmid, an autonomously replicating sequence (ARS), a promoter region, sequences for polyadenylation, and sequences for transcription termination. Preferably, yeast vectors include an origin of replication sequence and selectable marker. Suitable promoter sequences for yeast vectors include promoters for metallothionein, 3-phosphoglycerate kinase (Hitzeman et al., *J. Biol. Chem. 255*:2073, 1980) or other glycolytic enzymes (Hess et al., J. *Adv. Enzyme Reg. 7*:149, 1968; and Holland et al., *Biochem. 17*:4900, 1978), such as enolase, glyceraldehyde-3-phosphate dehydrogenase, hexokinase, pyruvate decarboxylase, phosphofructokinase, glucose-6-phosphate isomerase, 3-phosphoglycerate mutase, pyruvate kinase, triosephosphate isomerase, phosphoglucose isomerase, and glucokinase. Other suitable vectors and promoters for use in yeast expression are further described in Hitzeman, EPA-73,657.

Mammalian or insect host cell culture systems could also be employed to express recombinant CD40L polypeptides. Examples of suitable mammalian host cell lines include the COS-7 line of monkey kidney cells (ATCC CRL 1651) (Gluzman et al., *Cell 23*:175, 1981), L cells, C127 cells, 3T3 cells (ATCC CCL 163), Chinese hamster ovary (CHO) cells, HeLa cells, and BHK (ATCC CRL 10) cell lines. Suitable mammalian expression vectors include nontranscribed elements such as an origin of replication, a promoter sequence, an enhancer linked to the structural gene, other 5' or 3' flanking nontranscribed sequences, such as ribosome binding sites, a polyadenylation site, splice donor and acceptor sites, and transcriptional termination sequences.

Transcriptional and translational control sequences for mammalian host cell expression vectors may be excised from viral genomes. For example, commonly used mammalian cell promoter sequences and enhancer sequences are derived from Polyoma virus, Adenovirus 2, Simian Virus 40 (SV40), and human cytomegalovirus. DNA sequences derived from the SV40 viral genome, for example, SV40 origin, early and late promoter, enhancer, splice, and polyadenylation sites may be used to provide the other genetic elements required for expression of a structural gene sequence in a mammalian host cell. Viral early and late promoters are particularly useful because both are easily obtained from a viral genome as a fragment which may also contain a viral origin of replication (Fiers et al., *Nature* 273:113, 1978). Smaller or larger SV40 fragments may also be used, provided the approximately 250 bp sequence extending from the *Hind* III site toward the *Bgl* I site located in the SV40 viral origin of replication site is included.

Exemplary mammalian expression vectors can be constructed as disclosed by Okayama and Berg (*Mol. Cell. Biol. 3*:280, 1983). A useful high expression vector, PMLSV N1/N4, described by Cosman et al., *Nature 312*:768, 1984 has been deposited as ATCC 39890. Additional useful mammalian expression vectors are described in EP-A-0367566, and in U.S. Patent Application Serial No. 07/701,415, filed May 16, 1991. For expression of a type II protein extracellular region, such as CD40L, a heterologous signal sequence should be added, such as the signal sequence for interleukin-7 (IL-7) described in United States Patent 4,965,195, or the signal sequence for interleukin-2 receptor described in United States Patent Application 06/626,667 filed on July 2, 1984.

### Purification of Recombinant CD40L Polypeptides

CD40L polypeptides may be prepared by culturing transformed host cells under culture conditions necessary to express CD40L polypeptides. The resulting expressed polypeptides may then be purified from culture media or cell extracts. A CD40L polypeptide, if desired, may be concentrated using a commercially available protein concentration filter, for example, an Amicon or Millipore Pellicon ultrafiltration unit. Following the concentration step, the concentrate can be applied to a purification matrix such as a gel filtration medium. Alternatively, an anion exchange resin can be employed, for example, a matrix or substrate having pendant diethylaminoethyl (DEAE) groups. The matrices can be acrylamide, agarose, dextran, cellulose or other types commonly employed in protein purification. Alternatively, a cation exchange step can be employed. Suitable cation exchangers include various insoluble matrices comprising sulfopropyl or carboxymethyl groups. Sulfopropyl groups are preferred.

Finally, one or more reverse-phase high performance liquid chromatography (RP-HPLC) steps employing hydrophobic RP-HPLC media, (e.g., silica gel having pendant methyl or other aliphatic groups) can be employed to further purify CD40L. Some or all of the foregoing purification steps, in various combinations, can also be employed to provide a substantially homogeneous recombinant protein.

It is also possible to utilize an affinity column comprising CD40 ligand binding domain to affinity-purify expressed CD40L polypeptides. CD40L polypeptides can be removed from an affinity column in a high salt elution buffer and then dialyzed into a lower salt buffer for use.

Recombinant protein produced in bacterial culture is usually isolated by initial disruption of the host cells, centrifugation, extraction from cell pellets if an insoluble polypeptide, or from the supernatant fluid if a soluble polypeptide, followed by one or more concentration, salting-out, ion exchange, affinity purification or size exclusion chromatography steps. Finally, RP-HPLC can be employed for final purification steps. Microbial cells can be disrupted by any convenient method, including freeze-thaw cycling, sonication, mechanical disruption, or use of cell lysing agents.

Transformed yeast host cells are preferably employed to express CD40L as a secreted polypeptide. This simplifies purification. Secreted recombinant polypeptide from a yeast host cell fermentation can be purified by methods analogous to those disclosed by Urdal et al. (*J. Chromatog. 296*:171, 1984). Urdal et al. describe two sequential, reversed-phase HPLC steps for purification of recombinant human IL-2 on a preparative HPLC column.

### Administration of CD40L Compositions

The present invention provides therapeutic compositions comprising an effective amount of the CD40L mutein in a suitable diluent or carrier and methods of treating mammals using the compositions. For therapeutic use, the purified CD40L mutein is administered to a patient, preferably a human, for treatment in a manner appropriate to the indication. Thus, for example, CD40L mutein pharmaceutical compositions (for example, in the form of a soluble CD40L mutein comprising the tryptophan at amino acid 194) which is administered to achieve a desired therapeutic effect can be given by bolus injection, continuous infusion, sustained release from implants, or other suitable technique.

Typically, a CD40L mutein therapeutic agent will be administered in the form of a pharmaceutical composition comprising purified CD40L mutein in conjunction with physiologically acceptable carriers, excipients or diluents. Such carriers will be nontoxic to patients at the dosages and concentrations employed. Ordinarily, the preparation of such compositions entails combining a CD40L mutein with buffers, antioxidants such as ascorbic acid, low molecular weight (less than about 10 residues) polypeptides, proteins, amino acids, carbohydrates including glucose, sucrose or dextrans, chelating agents such as EDTA, glutathione and other stabilizers and excipients. Neutral buffered saline or saline mixed with conspecific serum albumin are exemplary appropriate diluents.

The following examples are intended to illustrate particular embodiments and not limit the scope of the invention.

### EXAMPLE 1

This example describes two solid-phase binding assays, the first of which, (a), can be used to asses the ability of trimeric CD40L to bind CD40, and the second of which, (b), is used to detect the presence of CD40L.

### (a) Quantitative CD40L ELISA

CD40/Fc is prepared and purified as described in WO 93/08207, and used to coat 96-well plates (Coming EasyWash ELISA plates, Corning, NY, USA). The plates are coated with 2.5 µg/well of CD40/Fc in PBS overnight at 4°C, and blocked with 1 % non-fat milk in PBS for 1 hour at room temperature. Samples to be tested are diluted in 10% normal goat serum in PBS, and 50 µl is added per well. A titration of unknown samples is run in duplicate, and a titration of reference standard of CD40L is run to generate a standard curve. The plates are incubated with the samples and controls for 45 minutes at room temperature, then washed four times with PBS. Second step reagent, rabbit anti-oligomerizing zipper, is added (50 µl/well, concentration approximately 2.5 µg/ml), and the plates are incubated at room temperature for 45 minutes. The plates are again washed as previously described, and goat F(ab')2 anti-rabbit IgG conjugated to horseradish peroxidase (Tago, Burlingame, CA, USA) is added. Plates are incubated for 45 minutes at room temperature, washed as described, and the presence of CD40L is detected by the addition of chromogen, tetramethyl benzidene (TMB; 100µl/well) for 15 minutes at room temperature. The chromogenic reaction is stopped by the addition of 100 µl/well 2N H₂SO₄, and the OD₄₅₀-OD₅₆₂ of the wells determined. The quantity of trimeric CD40L can be determined by comparing the OD values obtained with the unknown samples to the values generated for the standard curve. Values are expressed as the number of binding units per ml. A binding unit is roughly one ng of protein as estimated using a purified Fc fusion protein of the ligand as a standard. In this manner, the concentration and specific activity of several different batches of trimeric CD40L have been determined.

### (b) Qualitative Dot Blot

CD40L trimer (1 µl of crude supernatant or column fractions) is adsorbed to dry BA85/21 nitrocellulose membranes (Schleicher and Schuell, Keene, NH) and allowed to dry. The membranes are incubated in tissue culture dishes for one hour in Tris (0.05 M) buffered saline (0.15 M) pH 7.5 containing 1% w/v BSA to block nonspecific binding sites. At the end of this time, the membranes are washed three times in PBS, and rabbit anti-oligomerizing zipper antibody is added at an approximate concentration of 10 µg/ml in PBS containing 1% BSA, following which the membranes are incubated for one hour at room temperature. The membranes are again washed as described, and a horseradish peroxidase (HRP)-labeled antibody (such as goat anti-rabbit Ig; Southern Biotech, Birmingham, AL) at an approximate dilution of 1:1000 in PBS containing 1% BSA is added. After incubating for one hour at room temperature, the membranes are washed and chromogen (i.e. 4-chloronaphthol reagent, Kirkegard and Perry, Gaithersburg, MD) is added. Color is allowed to develop for ten minutes at room temperature, and the reaction is stopped by rinsing the membranes with water. The membranes are washed, and the presence of CD40L is determined by analyzing for the presence of a blue-black color. This assay was used to determine the presence or absence of trimeric CD40L in cell culture supernatant fluids and in purification column fractions. The assay further provides a semi-quantitative method of determining relative amounts of trimeric CD40L by comparing the intensity of the color in unknown samples to the intensity of known quantities of controls.

### EXAMPLE 2

This example describes construction of a human CD40L DNA construct to express trimeric CD40L in Chinese hamster ovary (CHO) cells. Trimeric CD40L contains a leader sequence, and a 33 amino acid sequence referred to as an oligomerizing zipper (SEQ ID NO:5), followed by the extracellular region of human CD40L from amino acid 51 to amino acid 261 (SEQ ID NO:1). The construct was prepared by cutting the appropriate DNA from a plasmid containing human CD40L, and ligating the DNA into the expression vector pCAVDHFR. The resultant construct was referred to as CAV/DHFR-CD40LT. pCAVDHFR includes regulatory sequences derived from cytomegalovirus, SV40, and Adenovirus 2, along with the gene for dihydrofolate reductase (DHFR), and allows random integration of a desired gene into host cell chromosomes. Expression of DHFR enables the DHFR- host cells to grow in media lacking glycine, hypoxanthine, and thymidine (GHT). A similar construct was also made for expression of murine CD40L timer in CHO cells. In addition to the leader and oligomerizing zipper sequences, the murine construct also contained a sequence encoding the octapeptide referred to as Flag® (described previously) between the trimerization domain ("leucine zipper" or oligomerizing zipper) and the extracellular region of murine CD40L. The nucleotide and amino acid sequence of the human CD40L-encoding DNA's are shown in SEQ ID NO's 7 and 8 respectively. Additional constructs can be prepared using standard methods. For example, vectors which incorporates dual promoters such as those described in U.S. Patent 4,656,134, or vectors employing enhancer sequences such as those described in U.S. Patent 4,937,190 or in Kaufman et al., *Nucl. Acids Res.* 19:4485, 1991, are also useful in preparing constructs for expression of CD40L in CHO cells.

The resulting ligation product was transfected into CHO cells using either Lipofectin® Reagent or Lipofectamine™ Reagent (Gibco BRL, Gaithersburg, MD). Both of these reagents are commercially available reagents used to form lipid-nucleic acid complexes (or liposomes) which, when applied to cultured cells, facilitate uptake of the nucleic acid into the cells. Cells which were transfected with the pCAVDHFR-CD40LT construct were selected in DMEM:F12 medium in the absence of GHT. Cells which were able to grow in the absence of GHT were tested for production of CD40L using a solid phase binding assay as described in Example 16. Results indicated that in this transfection system, Lipofectamine™ Reagent gave higher rates of successful transfection.

Approximately 160 clones were screened and two positive clones were identified and expanded for further study. Cells were passaged in GHT-free DMEM:F12 medium, and monitored for stability by assessing production of trimeric CD40L in the solid-phase binding assay described above. Based on these results, one clone was chosen which appeared to be stabley transfected with the CD40L DNA, and which produced and secreted approximately 1 µg/10⁶ cells/day of CD40L trimer. Additional constructs comprising other vectors and all or a portion of the DNA sequences described in this example can be used to prepare additional stabley transfected cell lines, substantially as described herein.

Once such stabley transfected cells were identified, large scale cultures of transfected cells were grown to accumulate supernatant containing trimeric CD40L. Suitable large-scale culture conditions include the use of bioreactors. Similar procedures were followed to produce CHO cell lines that secreted a trimeric murine CD40L at approximately 0.05 µg/10⁶ cells/day. CHO cells stabley transfected with either the human or murine CD40L construct, having acquired a DHFR gene from the pCAVDHFR plasmid, are resistant to methotrexate. Methotrexate can be added to the culture medium to amplify the number of copies of the CD40L trimer DNA in order to increase production of CD40L trimer.

### EXAMPLE 3

This example illustrates the binding affinities of several different CD40L constructs. Affinity experiments were conducted by biospecific interaction analysis (BIA) using a biosensor, an instrument that combines a biological recognition mechanism with a sensing device or transducer. An exemplary biosensor is BIAcore™, from Pharmacia Biosensor AB (Uppsala, Sweden; see Fägerstam L.G., *Techniques in Protein Chemistry II,* ed. J.J. Villafranca, Acad. Press, NY, 1991). BIAcore™ uses the optical phenomenon surface plasmon resonance (Kretschmann and Raether, Z *Naturforschung, Teil. A* 23:2135, 1968) to monitor the interaction of two biological molecules. Molecule pairs having affinity constants in the range 10⁵ to 10¹⁰ M⁻¹, and association rate constants in the range of 10³ to 10⁶ M⁻¹s⁻¹, are suitable for characterization with BIAcore™.

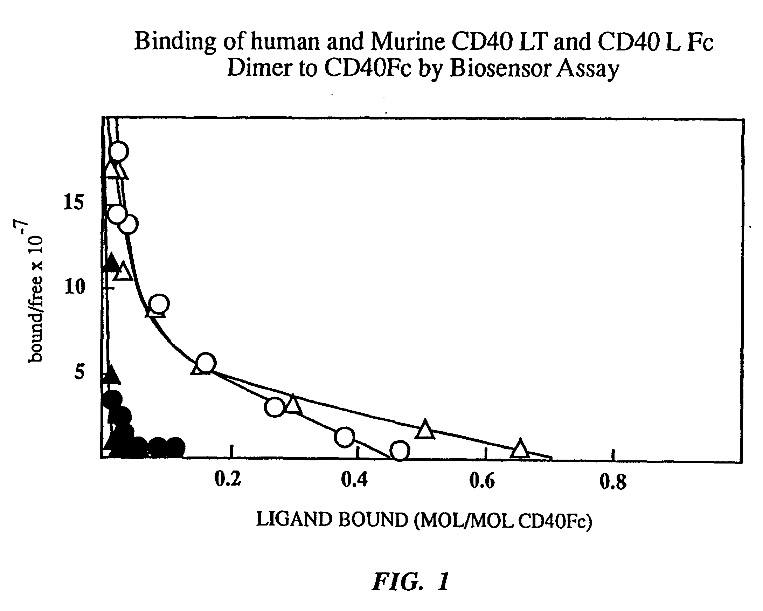

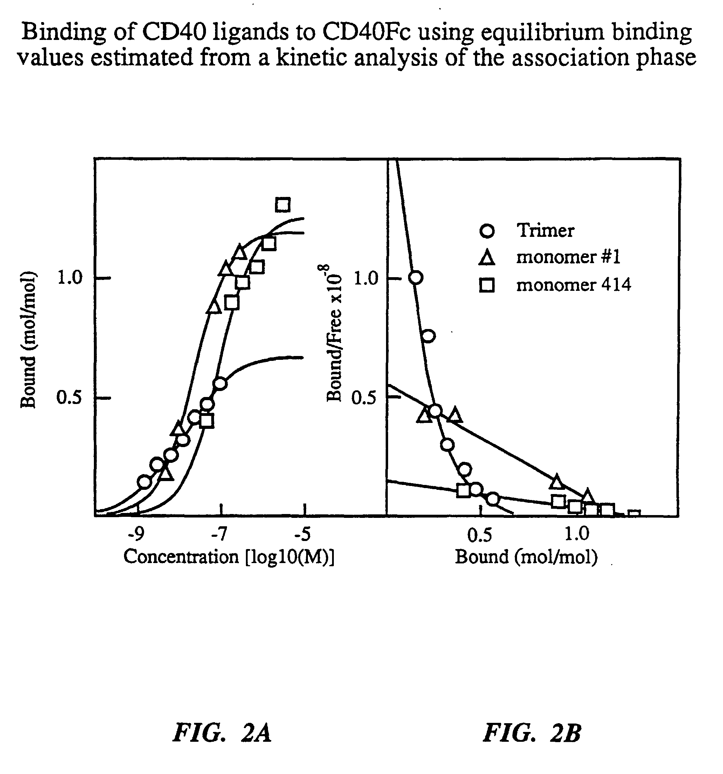

The biosensor chips were coated with goat anti-human IgG₁ Fc, which was used to bind CD40/Fc to the chip. The different constructs of CD40L were then added at increasing concentrations; the chip was regenerated between the different constructs by the addition of sodium hydroxide. Two separate experiments were performed. In the first, the binding of a dimeric human CD40L (CD40L/Fc), trimeric human CD40L (CD40L/"leucine zipper"), and dimeric and trimeric murine CD40L (prepared substantially as described for the human CD40L) were compared. In the second experiment, the binding of trimeric human CD40L was compared to the binding of two different preparations of monomeric human CD40L (comprising solely the portion of the extracellular domain most homologous to TNF). The resultant data were analyzed to determine the affinity and association rate constants of the different CD40L constructs. Results are shown in Table 1 below, and in Figures 1 and 2.

**Table 1**

| : Binding of CD40L to CD40/Fc | | | | |
|---|---|---|---|---|
| | Site 1 (mol/mol CD40/Fc) | K₁ (M⁻¹) | Site2 (mol/mol CD40/Fc) | K₂ (M⁻¹) |
| Human Trimer | 0.04 ±0.02 | 5 ± 3 x 10⁹ | 0.68 ± 0.07 | 7.5 ± 2.5 x 10⁷ |
| Human Dimer | 0.013 ± 0.002 | 1.6 ± 0.7 x 10¹¹ | 0.049 ± 0.004 | 7.0 ± 2.1 x 10⁸ |
| Murine Trimer | 0.02 ± 0.003 | 6 ± 3 x 10¹⁰ | 0.44 ± 0.02 | 1.6 ± 0.2 x 10⁸ |
| Murine Dimer | 0.05 ± 0.02 | 7 ± 4 x 10⁹ | 0.14 ± 0.23 | 4.0 ± 1.0 x 10⁷ |
| Human Trimer | 0.26 | 6.6 x 10⁸ | 0.41 | 2.6 x 10⁷ |
| Monomer #1 | Not Detected | Not Detected | 1.20 | 4.6 x 107 |
| Monomer #2 | Not Detected | Not Detected | 1.27 | 1.1 x 10⁷ |

Analysis of the data indicated that a CD40L monomer comprising solely the portion of the extracellular domain most homologous to TNF was capable of binding CD40, although with somewhat lower affinity than oligomeric CD40L. An analysis of the ratio of binding in the second experiment demonstrated that there are twice as many CD40L monomer units bound per CD40/Fc molecule as trimeric CD40L, confirming that two monomers of CD40L bind one CD40/Fc dimer and one trimeric CD40L binds one CD40/Fc dimer.

### Example 4

This example illustrates preparation of a number of muteins of a CD40 ligand/zipper domain fusion protein. Mutations for constructs to be expressed in yeast (mutants 14, 18, 32, 41, 43, 10PP and 18PP) were generated by PCR misincorporation (Mulrad et al *Yeast* 8:79, 1992), and selected based on an apparent increase in secretion as improved secretion mutants. Mutants 14, 18, 32, 41, and 43 were isolated in *S. cerevisiae.* Mutants 10PP and 18PP were isolated in *P. pastoris.* Mutations for constructs to be expressed in mammalian cells (FL194.W, 194.W, LZ12V, 215.T, 255.F, and 194.S) were also prepared using PCR, and were either the result of site-directed mutagenesis or were the random product of PCR. The types of mutations obtained and their effect on activity (ability to bind CD40 in a solid phase binding assay substantially as described in Example 1) are shown in Table 2 below.

**Table 2:**

| Mutations present in the CD40 ligand/zipper domain fusion protein | | | | |
|---|---|---|---|---|
| Mutant No.: | Zipper Domain Mutation^{a} | CD40L Domain Mutations^{b} | Activity | Type of Mutant |
| 14 | I12N | K260N | + | random mutant |
| 18 | L13P | A130P, R181Q | + | random mutant |
| 32 | I12N | Q121P | + | random mutant |
| 41 | I5M, I16T | NA | + | random mutant |
| 43 | I16N | T134S, K164I, Q186L, N210S | + | random mutant |
| 10PP | I9N, K27R | NA^{d} | + | random mutant |
| 18PP | L13P | NA | + | random mutant |
| LZ12.V | I12V | Deletion of aa 1-112 | + | PCR; random |
| 215.T | NA | Deletion of aa 1-112; A215T | + | PCR; random |
| 255.F | NA | Deletion of aa 1-112; S255F | - | PCR; site-directed |
| FL194W | NA | C194W | + | PCR; site-directed |
| 194.W | NA | Deletion of aa 1-112; C194W | + | PCR; site-directed |
| 194.S | NA | Deletion of aa 1-112; C194S | ND^{e} | PCR; site-directed |
| 194.A | NA | Deletion of aa 1-112; C194A | ND^{e} | PCR; site-directed |
| 194.D | NA | Deletion of aa 1-112; C194D | ND^{e} | PCR; site-directed |
| 194.K | NA | Deletion of aa 1-112; C194K | ND^{e} | PCR; site-directed |

| | | | | |
|---|---|---|---|---|
| a: Mutations are given as the residue present in the native peptide, the residue number, and the residue present in the mutein. Residue numbers for zipper domain mutations are relative to SEQ ID NO:5. b: Residue numbers for mutations in the CD40L domain are relative to SEQ ID NO:1. c: Mutant 10PP also contained mutations in regions other than CD40L domain or the zipper domain (T-4S, D-2P, relative to SEQ ID NO:7). | | | | |
| d: Not applicable | | | | |
| e: Not done | | | | |

Mutant 18PP had only a single mutation in the molecule, which was sufficient to affect secretion in yeast. Mutant 41 had two mutations, both of which were in the isoleucine residues of the zipper domain. The mutations in the zipper improve secretion from yeast without apparent effect on activity. Mutant 194.W was expressed in yeast cells and purified either by a combination of ion exchange chromatography steps (194.W (c)) or by affinity chromatography (194.W (a)) using a monoclonal antibody that binds the oligomerizing zipper moiety. oligomerizing zipper moiety. The yeast-expressed mutant (194.W) exhibited greater affinity for CD40 in a biosensor assay performed substantially as described in Example 3, and exhibited greater biological activity than wild type CD40 ligand/zipper domain fusion protein (WT) expressed in yeast, in a B cell proliferation assay. These results are shown in Table 3.

**Table 3:**

| Comparison of WT and 194.W for Receptor Binding and B-cell Proliferation | | | |
|---|---|---|---|
| | Affinity | B-cell proliferation (U/µg^{a}) | |
| | Kₐ (M⁻¹) | Experiment 1 | Experiment 2^{d} |
| WT | 7.7 x10⁷ | 77^{b} | 15 |
| 194.W (c) | 1.8 x 10⁹ | 171 | 116 |
| 194.W (a) | ND^{c} | ND | 161 |

| | | | |
|---|---|---|---|
| a: A unit (U) is the concentration that induces half-maximal proliferation | | | |
| b: Average from two independent preparations | | | |
| c: Not done | | | |
| d: Average of two assays | | | |

Moreover, FL194W expressed in mammalian cells also demonstrated higher binding that WT CD40L in a semi-quantitative western blot analysis.

Additional constructs were prepared by substituting the lung surfactant protein D (SPD) trimerization domain (SEQ ID NO:6; Hoppe, et al., *FEBS Letters* 344:191, 1994) in place of the trimer-forming zipper of SEQ ID NO:5. This construct is. expressed in *S. cerevlsiae* and in mammalian cells at low levels. Activity is determined as described previously; various mutants based on such constructs can also be prepared to optimize secretion or other product characteristics, as described above.

### EXAMPLE 5

This example illustrates the binding affinities of several different CD40L constructs. Affinity experiments were conducted by biospecific interaction analysis (BIA), substantially as described in Example 3, using two of the constructs described in Example 4. Results presented in Table 4 represent the average values obtained for two different preparations of wild-type (WT) CD40LT, and three different preparations of mutein (194.W). Results are shown below.

**Table 4:**

| Binding of CD40LT to CD40/Fc | | |
|---|---|---|
| | K₁ (M⁻¹) | K₂ (M⁻¹) |
| CD40LT WT | 7.4 ± 3.6 x 10⁸ | 3.0 ± 0.4 x 10⁷ |
| CD40LT 194.W | 9.7 ± 3.6 x 10⁹ | 5.4 ± 1.5 x 10⁸ |

These results confirm that the CD40LT 194.W has a higher affinity for CD40/Fc than does the wild type CD40LT.

## Claims

1. An isolated DNA encoding a CD40L mutein, selected from the group consisting of:
(a) a DNA that encodes a polypeptide having an amino acid sequence as set forth in SEQ ID NO: 2 wherein a cysteine at amino acid 194 is replaced with tryptophan and
(b) a DNA that encodes a polypeptide that is a fragment of the mutein (a) comprising tryptophan an amino acid 194, and which polypeptide binds CD40.

2. The isolated DNA of claim 1 that encodes a polypeptide selected from the group consisting of polypeptides comprising amino acids 1 through 261, 35 through 261, 34 through 225, 113 through 261, 113 through 225, 120 through 261, or 120 through 225 of SEQ ID NO: 2.

3. The isolated DNA according to claim 2 which further comprises a DNA encoding an oligomerizing zipper having an amino acid sequence represented by SEQ ID NO: 5.

4. The isolated DNA of claim 3, wherein the DNA encodes a fusion protein comprising the oligomerizing zipper fused to the amino-proximal end of a fragment of the extracellular domain of CD40L consisting of amino acids 113 through 261 of SEQ ID NO:2.

5. A recombinant expression vector comprising a DNA according to anyone of claims 1 to 4.

6. A host cell transformed or transfected with a recombinant expression vector according to claim 5.

7. A process for preparing a CD40L polypeptide, comprising culturing a host cell according to claim 6 under conditions promoting expression and recovering CD40L polypeptide from the culture.

8. A CD40L polypeptide encoded by a DNA according to any one of claims 1 through 4.

9. A pharmaceutical composition comprising a polypeptide of claim 8 together with a carrier, excipient or diluent.

10. A CD40L polypeptide according to claim 8 or a pharmaceutical composition according to claim 9 for use in inducing immunoglobulin secretion.

11. A CD40L polypeptide according to claim 8 or a pharmaceutical composition according to claim 9 for use in inducing B-cell proliferation.

## Patentansprüche

1. Isolierte DNA, welche ein CD40L-Mutein codiert, welche ausgewählt ist aus der Gruppe bestehend aus:
(a) einer DNA, welche ein Polypeptid codiert, das eine Aminosäuresequenz gemäß SEQ-ID Nr. 2 besitzt, wobei ein Cystein an der Aminosäure 194 durch Tryptophan ersetzt ist, und
(b) einer DNA, welche ein Polypeptid codiert, bei dem es sich um ein Fragment des Muteins (a) handelt, das Tryptophan an der Aminosäure 194 enthält, und welche das Polypeptid CD40 bindet.

2. Isolierte DNA nach Anspruch 1, welche ein Polypeptid codiert, das aus der Gruppe bestehend aus Polypeptiden ausgewählt wird, welche die Aminosäuren 1 bis 261, 35 bis 261, 34 bis 225, 113 bis 261, 113 bis 225, 120 bis 261, oder 120 bis 225 der SEQ-ID Nr. 2 enthält.

3. Isolierte DNA nach Anspruch 2, welche weiters eine DNA enthält, die einen oligomerisierenden Zipper codiert, der durch eine SEQ-ID Nr. 5 repräsentierte Aminosäuresequenz besitzt.

4. Isolierte DNA nach Anspruch 3, wobei die DNA ein Fusionsprotein codiert, umfassend den oligomerisierenden Reißverschluss, der mit dem amino-proximalen Ende eines Fragments der extrazellulären Domäne von CD40L, bestehend aus den Aminosäuren 113 bis 261 der SEQ-ID Nr. 2, verschmolzen ist.

5. Rekombinierter Expressionsvektor, der eine DNA gemäß einem der Ansprüche 1 bis 4 enthält.

6. Wirtszelle, welche mit einem rekombinierten Expressionsvektor nach Anspruch 5 transformiert oder transfektiert ist.

7. Verfahren zur Herstellung eines CD40L-Polypeptids, welches die Züchtung einer Wirtszelle nach Anspruch 6 unter Bedingungen aufweist, welche die Expression und Wiedergewinnung des CD40L-Polypeptids aus der Kultur fördern.

8. CD40L-Polypeptid, das von einer DNA nach einem der Ansprüche 1 bis 4 codiert wurde.

9. Pharmazeutische Zusammensetzung, die ein Polypeptid nach Anspruch 8, zusammen mit einer Trägersubstanz, einem Arzneimittelträger oder einem Verdünnungsmittel aufweist.

10. CD40L-Polypeptid nach Anspruch 8 oder eine pharmazeutische Zusammensetzung nach Anspruch 9 für die Verwendung bei der Induktion einer Immunglobulinsekretion.

11. CD40L-Polypeptid nach Anspruch 8 oder eine pharmazeutische Zusammensetzung nach Anspruch 9 für die Verwendung bei der Induktion einer B-Zellen-Proliferation.

## Revendications

1. ADN isolé codant pour une protéine mutée de CD40L, sélectionnée parmi le groupe comprenant :
(a) un ADN qui code pour un polypeptide présentant une séquence d'acides aminés comme décrit à la SEQ ID n° 2, dans laquelle une cystéine au niveau de l'acide aminé 194 est remplacée par un tryptophane et
(b) un ADN qui code pour un polypeptide qui est un fragment de la protéine mutée (a) comprenant un tryptophane comme acide aminé 194 et lequel polypeptide lie le CD40.

2. ADN isolé suivant la revendication 1, qui code pour un polypeptide sélectionné parmi le groupe comprenant des polypeptides comprenant les acides aminés 1 à 261, 35 à 261, 34 à 225, 113 à 261, 113 à 225, 120 à 261 ou 120 à 225 de la SEQ ID n° 2.

3. ADN isolé suivant la revendication 2, qui comprend en outre un ADN codant pour un domaine à glissière formant un oligomère présentant une séquence d'acides aminés représentée par la SEQ ID n° 5.

4. ADN isolé suivant la revendication 3, dans lequel l'ADN code pour une protéine de fusion comprenant le domaine à glissière formant un oligomère fusionné à l'extrémité du côté amino d'un fragment du domaine extracellulaire du CD40L comprenant les acides aminés 113 à 261 de la SEQ ID n° 2.

5. Vecteur recombinant d'expression comprenant un ADN suivant l'une quelconque des revendications 1 à 4.

6. Cellule hôte transformée ou transfectée par un vecteur recombinant d'expression suivant la revendication 5.

7. Procédé de préparation d'un polypeptide CD40L comprenant la mise en culture d'une cellule hôte suivant la revendication 6 dans des conditions promouvant l'expression et la récupération d'un polypeptide CD40L à partir de la culture.

8. Polypeptide CD40L codé par un ADN suivant l'une quelconque des revendications 1 à 4.

9. Composition pharmaceutique comprenant un polypeptide suivant la revendication 8, conjointement avec un vecteur, un excipient ou un diluant.

10. Polypeptide CD40L suivant la revendication 8, ou composition pharmaceutique suivant la revendication 9 pour une utilisation pour l'induction d'une sécrétion d'immunoglobulines.

11. Polypeptide CD40L suivant la revendication 8 ou composition pharmaceutique suivant la revendication 9, pour une utilisation pour l'induction d'une prolifération de lymphocytes B.


### SEQUENCE LISTING

(1) GENERAL INFORMATION:
   (i) APPLICANT: IMMUNEX CORPORATION
   (ii) TITLE OF INVENTION: NOVEL CD40L MUTEIN
   (iii) NUMBER OF SEQUENCES: 8
   (iv) CORRESPONDENCE ADDRESS:
      (A) ADDRESSEE: IMMUNEX CORPORATION
      (B) STREET: 51 UNIVERSITY STREET
      (C) CITY: SEATTLE
      (D) STATE: WASHINGTON
      (E) COUNTRY: USA
      (F) ZIP: 98101
   (v) COMPUTER READABLE FORM:
      (A) MEDIUM TYPE: Floppy disk
      (B) COMPUTER: Apple Macintosh
      (C) OPERATING SYSTEM: Apple Operating System 7.5.3
      (D) SOFTWARE: Microsoft Word for Apple, version 6.0.1a
   (vi) CURRENT APPLICATION DATA:
      (A) APPLICATION NUMBER:
      (B) FILING DATE: : 06 JUN 1996
      (C) CLASSIFICATION:
   (vii) PRIOR APPLICATION DATA:
      (A) APPLICATION NUMBER: USSN 08/477,733
      (B) FILING DATE: 07 JUN 1995
      (C) CLASSIFICATION:
   (vii) PRIOR APPLICATION DATA:
      (A) APPLICATION NUMBER: USSN 08/484,624
      (B) FILING DATE: 07 JUN 1995
      (C) CLASSIFICATION:
   (viii) ATTORNEY/AGENT INFORMATION:
      (A) NAME: Perkins, Patricia Anne
      (B) REGISTRATION NUMBER: 34,693
      (C) REFERENCE/DOCKET NUMBER: 2802-DWO
   (ix) TELECOMMUNICATION INFORMATION:
      (A) TELEPHONE: 2065870430
      (B) TELEFAX: 2062330644
(2) INFORMATION FOR SEQ ID NO:1:
   (i) SEQUENCE CHARACTERISTICS:
      (A) LENGTH: 840 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: NO
   (vi) ORIGINAL SOURCE:
      (A) ORGANISM: Homo sapiens
   (vii) IMMEDIATE SOURCE:
      (B) CLONE: CD40L
   (ix) FEATURE:
      (A) NAME/KEY: CDS
      (B) LOCATION: 46..831
   (xi) SEQUENCE DESCRIPTION: SEQ ID NO:1:
TGCCACCTTC TCTGCCAGAA GATACCATTT CAACTTTAAC ACAGC ATG ATC GAA 54
   ACA TAC AAC CAA ACT TCT CCC CGA TCT GCG GCC ACT GGA CTG CCC ATC    102
   AGC ATG AAA ATT TTT ATG TAT TTA CTT ACT GTT TTT CTT ATC ACC CAG    150
   ATG ATT GGG TCA GCA CTT TTT GCT GTG TAT CTT CAT AGA AGG TTG GAC    198
   AAG ATA GAA GAT GAA AGG AAT CTT CAT GAA GAT TTT GTA TTC ATG AAA    246
   ACG ATA CAG AGA TGC AAC ACA GGA GAA AGA TCC TTA TCC TTA CTG AAC    294
   TGT GAG GAG ATT AAA AGC CAG TTT GAA GGC TTT GTG AAG GAT ATA ATG    342
   TTA AAC AAA GAG GAG ACG AAG AAA GAA AAC AGC TTT GAA ATG CAA AAA    390
   GGT GAT CAG AAT CCT CAA ATT GCG GCA CAT GTC ATA AGT GAG GCC AGC    438
   AGT AAA ACA ACA TCT GTG TTA CAG TGG GCT GAA AAA GGA TAC TAC ACC    486
   ATG AGC AAC AAC TTG GTA ACC CTG GAA AAT GGG AAA CAG CTG ACC GTT    534
   AAA AGA CAA GGA CTC TAT TAT ATC TAT GCC CAA GTC ACC TTC TGT TCC    582
   AAT CGG GAA GCT TCG AGT CAA GCT CCA TTT ATA GCC AGC CTC TGC CTA    630
   AAG TCC CCC GGT AGA TTC GAG AGA ATC TTA CTC AGA GCT GCA AAT ACC     678
   CAC AGT TCC GCC AAA CCT TGC GGG CAA CAA TCC ATT CAC TTG GGA GGA     726
   GTA TTT GAA TTG CAA CCA GGT GCT TCG GTG TTT GTC AAT GTG ACT GAT     774
   CCA AGC CAA GTG AGC CAT GGC ACT GGC TTC ACG TCC TTT GGC TTA CTC     822
   AAA CTC TGAACAGTGT CA                                               840
(2) INFORMATION FOR SEQ ID NO:2:
   (i) SEQUENCE CHARACTERISTICS:
      (A) LENGTH: 261 amino acids
      (B) TYPE: amino acid
      (D) TOPOLOGY: linear
   (ii) MOLECULE TYPE: protein
   (xi) SEQUENCE DESCRIPTION: SEQ ID NO:2:
(2) INFORMATION FOR SEQ ID NO:3:
   (i) SEQUENCE CHARACTERISTICS:
      (A) LENGTH: 519 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: NO
   (vi) ORIGINAL SOURCE:
      (A) ORGANISM: HUMAN
   (vii) IMMEDIATE SOURCE:
      (B) CLONE: CD40 EXTRACELLULAR REGION
   (xi) SEQUENCE DESCRIPTION: SEQ ID NO:3:
   CAGAACCACC CACTGCATGC AGAGAAAAAC AGTACCTAAT AAACAGTCAG TGCTGTTCTT    60
   TGTGCCAGCC AGGACAGAAA CTGGTGAGTG ACTGCACAGA GTTCACTGAA ACGGAATGCC   120
   TTCCTTGCGG TGAAAGCGAA TTCCTAGACA CCTGGAACAG AGAGACACAC TGCCACCAGC   180
   ACAAATACTG CGACCCCAAC CTAGGGCTTC GGGTCCAGCA GAAGGGCACC TCAGAAACAG   240
   ACACCATCTG CACCTGTGAA GAAGGCTGGC ACTGTACGAG TGAGGCCTGT GAGAGCTGTG   300
   TCCTGCACCG CTCATGCTCG CCCGGCTTTG GGGTCAAGCA GATTGCTACA GGGGTTTCTG   360
   ATACCATCTG CGAGCCCTGC CCAGTCGGCT TCTTCTCCAA TGTGTCATCT GCTTTCGAAA   420
   AATGTCACCC TTGGACAAGC TGTGAGACCA AAGACCTGGT TGTGCAACAG GCAGGCACAA   480
   ACAAGACTGA TGTTGTCTGT GGTCCCCAGG ATCGGCTGA                          519
(2) INFORMATION FOR SEQ ID NO:4:
   (i) SEQUENCE CHARACTERISTICS:
      (A) LENGTH: 740 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (iv) ANTI-SENSE: NO
   (vi) ORIGINAL SOURCE:
      (A) ORGANISM: HUMAN
   (vii) IMMEDIATE SOURCE:
      (B) CLONE: IgG1 Fc
   (xi) SEQUENCE DESCRIPTION: SEQ ID NO:4:
   CGGTACCGCT AGCGTCGACA GGCCTAGGAT ATCGATACGT AGAGCCCAGA TCTTGTGACA   60
   AAACTCACAC ATGCCCACCG TGCCCAGCAC CTGAACTCCT GGGGGGACCG TCAGTCTTCC  120
   TCTTCCCCCC AAAACCCAAG GACACCCTCA TGATCTCCCG GACCCCTGAG GTCACATGCG  180
   TGGTGGTGGA CGTGAGCCAC GAAGACCCTG AGGTCAAGTT CAACTGGTAC GTGGACGGCG  240
   TGGAGGTGCA TAATGCCAAG ACAAAGCCGC GGGAGGAGCA GTACAACAGC ACGTACCGGG  300
   TGGTCAGCGT CCTCACCGTC CTGCACCAGG ACTGGCTGAA TGGCAAGGAC TACAAGTGCA  360
   AGGTCTCCAA CAAAGCCCTC CCAGCCCCCA TGCAGAAAAC CATCTCCAAA GCCAAAGGGC  420
   AGCCCCGAGA ACCACAGGTG TACACCCTGC CCCCATCCCG GGATGAGCTG ACCAAGAACC  480
   AGGTCAGCCT GACCTGCCTG GTCAAAGGCT TCTATCCCAG GCACATCGCC GTGGAGTGGG  540
   AGAGCAATGG GCAGCCGGAG AACAACTACA AGACCACGCC TCCCGTGCTG GACTCCGACG  600
   GCTCCTTCTT CCTCTACAGC AAGCTCACCG TGGACAAGAG CAGGTGGCAG CAGGGGAACG  660
   TCTTCTCATG CTCCGTGATG CATGAGGCTC TGCACAACCA CTACACGCAG AAGAGCCTCT  720
   CCCTGTCTCC GGGTAAATGA                                               740
(2) INFORMATION FOR SEQ ID NO:5:
   (i) SEQUENCE CHARACTERISTICS:
      (A) LENGTH: 33 amino acids
      (B) TYPE: amino acid
      (D) TOPOLOGY: linear
   (ii) MOLECULE TYPE: peptide
   (xi) SEQUENCE DESCRIPTION: SEQ ID NO:5:
   Arg Met Lys Gln Ile Glu Asp Lys Ile Glu Glu Ile Leu Ser Lys Ile
    1               5                  10                  15
   Tyr His Ile Glu Asn Glu Ile Ala Arg Ile Lys Lys Leu Ile Gly Glu
               20                  25                  30
   Arg
(2) INFORMATION FOR SEQ ID NO:6:
   (i) SEQUENCE CHARACTERISTICS:
      (A) LENGTH: 27 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (xi) SEQUENCE DESCRIPTION: SEQ ID NO:6:
   Pro Asp Val Ala Ser Leu Arg Gln Gln Val Glu Ala Leu Gln Gly Gln
    1               5                   10                  15
   Val Gln His Leu Gln Ala Ala Phe Ser Gln Tyr
               20                  25
(2) INFORMATION FOR SEQ ID NO:7:
   (i) SEQUENCE CHARACTERISTICS:
      (A) LENGTH: 929 base pairs
      (B) TYPE: nucleic acid
      (C) STRANDEDNESS: single
      (D) TOPOLOGY: linear
   (ii) MOLECULE TYPE: cDNA
   (vi) ORIGINAL SOURCE:
      (A) ORGANISM: Human CD40L trimer
   (ix) FEATURE:
      (A) NAME/KEY: sig_peptide
      (B) LOCATION: 65..142
   (ix) FEATURE:
      (A) NAME/KEY: CDS
      (B) LOCATION: 65..886
   (ix) FEATURE:
      (A) NAME/KEY: mat_peptide
      (B) LOCATION: 143..886
   (xi) SEQUENCE DESCRIPTION: SEQ ID NO:7:
   TGAGCGAGTC CGCATCGACG GATCGGAAAA CCTCTCCGAG GTACCTATCC CGGGGATCCC    60
   CACC ATG TTC CAT GTT TCT TTT AGA TAT ATC TTT GGA ATT CCT CCA CTG   109
        Met Phe His Val Ser Phe Arg Tyr Ile Phe Gly Ile Pro Pro Leu
        -26 -25                 -20                 -15
   ATC CTT GTT CTG CTG CCT GTC ACT AGT TCT GAC CGT ATG AAA CAG ATA    157
   Ile Leu Val Leu Leu Pro Val Thr Ser Ser Asp Arg Met Lys Gln Ile
       -10                 -5                  1               5
   GAG GAT AAG ATC GAA GAG ATC CTA AGT AAG ATT TAT CAT ATA GAG AAT    205
   Glu Asp Lys Ile Glu Glu Ile Leu Ser Lys Ile Tyr His Ile Glu Asn
                   10                  15                  20
   GAA ATC GCC CGT ATC AAA AAG CTG ATT GGC GAG CGG ACT AGT TCT GAC    253
   Glu Ile Ala Arg Ile Lys Lys Leu Ile Gly Glu Arg Thr Ser Ser Asp
               25                  30                  35
   AAG ATA GAA GAT GAA AGG AAT CTT CAT GAA GAT TTT GTA TTC ATG AAA    301
   Lys Ile Glu Asp Glu Arg Asn Leu His Glu Asp Phe Val Phe Met Lys
           40                  45                  50
   ACG ATA CAG AGA TGC AAC ACA GGA GAA AGA TCC TTA TCC TTA CTG AAC    349
   Thr Ile Gln Arg Cys Asn Thr Gly Glu Arg Ser Leu Ser Leu Leu Asn
       55                  60                  65
   TGT GAG GAG ATT AAA AGC CAG TTT GAA GGC TTT GTG AAG GAT ATA ATG    397
   Cys Glu Glu Ile Lys Ser Gln Phe Glu Gly Phe Val Lys Asp Ile Met
   70                  75                  80                  85
   TTA AAC AAA GAG GAG ACG AAG AAA GAA AAC AGC TTT GAA ATG CAA AAA    445
   Leu Asn Lys Glu Glu Thr Lys Lys Glu Asn Ser Phe Glu Met Gln Lys
                   90                  95                  100
   GGT GAT CAG AAT CCT CAA ATT GCG GCA CAT GTC ATA AGT GAG GCC AGC    493
   Gly Asp Gln Asn Pro Gln Ile Ala Ala His Val Ile Ser Glu Ala Ser
               105                 110                 115
   AGT AAA ACA ACA TCT GTG TTA CAG TGG GCT GAA AAA GGA TAC TAC ACC    541
   Ser Lys Thr Thr Ser Val Leu Gln Trp Ala Glu Lys Gly Tyr Tyr Thr
           120                 125                 130
   ATG AGC AAC AAC TTG GTA ACC CTG GAA AAT GGG AAA CAG CTG ACC GTT    589
   Met Ser Asn Asn Leu Val Thr Leu Glu Asn Gly Lys Gln Leu Thr Val
       135                 140                 145
   AAA AGA CAA GGA CTC TAT TAT ATC TAT GCC CAA GTC ACC TTC TGT TCC    637
   Lys Arg Gln Gly Leu Tyr Tyr Ile Tyr Ala Gln Val Thr Phe Cys Ser
   150                 155                 160                 165
   AAT CGG GAA GCT TCG AGT CAA GCT CCA TTT ATA GCC AGC CTC TGC CTA    685
   Asn Arg Glu Ala Ser Ser Gln Ala Pro Phe Ile Ala Ser Leu Cys Leu
                   170                 175                 180
   AAG TCC CCC GGT AGA TTC GAG AGA ATC TTA CTC AGA GCT GCA AAT ACC    733
   Lys Ser Pro Gly Arg Phe Glu Arg Ile Leu Leu Arg Ala Ala Asn Thr
               185                 190                 195
   CAC AGT TCC GCC AAA CCT TGC GGG CAA CAA TCC ATT CAC TTG GGA GGA    781
   His Ser Ser Ala Lys Pro Cys Gly Gln Gln Ser Ile His Leu Gly Gly
           200                 205                 210
   GTA TTT GAA TTG CAA CCA GGT GCT TCG GTG TTT GTC AAT GTG ACT GAT      829
   Val Phe Glu Leu Gln Pro Gly Ala Ser Val Phe Val Asn Val Thr Asp
       215                 220                 225
   CCA AGC CAA GTG AGC CAT GGC ACT GGC TTC ACG TCC TTT GGC TTA CTC      877
   Pro Ser Gln Val Ser His Gly Thr Gly Phe Thr Ser Phe Gly Leu Leu
   230                 235                 240                 245
   AAA CTC TGAGCGGCCG CTACAGATGA ATAATAAGCA TGTTTGGATT CCTCAA           929
   Lys Leu
(2) INFORMATION FOR SEQ ID NO:8:
   (i) SEQUENCE CHARACTERISTICS:
      (A) LENGTH: 273 amino acids
      (B) TYPE: amino acid
      (D) TOPOLOGY: linear
   (ii) MOLECULE TYPE: protein
   (xi) SEQUENCE DESCRIPTION: SEQ ID NO:8:
   Met Phe His Val Ser Phe Arg Tyr Ile Phe Gly Ile Pro Pro Leu Ile
   -26 -25                 -20                 -15
   Leu Val Leu Leu Pro Val Thr Ser Ser Asp Arg Met Lys Gln Ile Glu
   -10                  -5                   1               5
   Asp Lys Ile Glu Glu Ile Leu Ser Lys Ile Tyr His Ile Glu Asn Glu
               10                  15                  20
   Ile Ala Arg Ile Lys Lys Leu Ile Gly Glu Arg Thr Ser Ser Asp Lys
           25                  30                  35
   Ile Glu Asp Glu Arg Asn Leu His Glu Asp Phe Val Phe Met Lys Thr
       40                  45                  50
   Ile Gln Arg Cys Asn Thr Gly Glu Arg Ser Leu Ser Leu Leu Asn Cys
    55                  60                  65                  70
   Glu Glu Ile Lys Ser Gln Phe Glu Gly Phe Val Lys Asp Ile Met Leu
                    75                  80                  85
   Asn Lys Glu Glu Thr Lys Lys Glu Asn Ser Phe Glu Met Gln Lys Gly
               90                  95                 100
   Asp Gln Asn Pro Gln Ile Ala Ala His Val Ile Ser Glu Ala Ser Ser
           105                 110                 115
   Lys Thr Thr Ser Val Leu Gln Trp Ala Glu Lys Gly Tyr Tyr Thr Met
       120                 125                 130
   Ser Asn Asn Leu Val Thr Leu Glu Asn Gly Lys Gln Leu Thr Val Lys
   135                 140                 145                 150
   Arg Gln Gly Leu Tyr Tyr Ile Tyr Ala Gln Val Thr Phe Cys Ser Asn
                   155                 160                 165
   Arg Glu Ala Ser Ser Gln Ala Pro Phe Ile Ala Ser Leu Cys Leu Lys
               170                 175                 180
   Ser Pro Gly Arg Phe Glu Arg Ile Leu Leu Arg Ala Ala Asn Thr His
           185                 190                 195
   Ser Ser Ala Lys Pro Cys Gly Gln Gln Ser Ile His Leu Gly Gly Val
       200                 205                 210
   Phe Glu Leu Gln Pro Gly Ala Ser Val Phe Val Asn Val Thr Asp Pro
   215                 220                 225                 230
   Ser Gln Val Ser His Gly Thr Gly Phe Thr Ser Phe Gly Leu Leu Lys
                   235                 240                 245
   Leu